# EUROPEAN PATENT APPLICATION

(11) **EP 4 477 642 A1**
(43) Date of publication of application: **18.12.2024**
(21) Application number: 23752886.4
(22) Date of filing: 08.02.2023
(51) Int. Cl.: C07C 49/603, A61K 31/122, A61P 13/02, A61P 25/00, A61P 25/04, A61P 29/02

(54) **CRYSTAL FORM OF CYCLOHEXENONE COMPOUND**

(30) Priority: 09.02.2022 JP 2022019058
(71) Applicant: Taiho Pharmaceutical Co., Ltd., Chiyoda-ku, Tokyo 101-8444 (JP)
(72) Inventor: SHIGENO, Kazuhiko, Tsukuba-shi, Ibaraki 300-2611 (JP); NAKAMURA, Hiroyuki, Tsukuba-shi, Ibaraki 300-2611 (JP)
(74) Representative: Hoffmann Eitle
(86) International application number: PCT/JP2023/004145
(87) International publication number: WO 2023/153422

(57) **Abstract**

An object of the present disclosure is to provide a crystalline form of 3-(15-methoxypentadecyl)-2,4,4-trimethylcyclohex-2-en-1-one, the crystalline form having excellent stability and being preferable in terms of production. The present disclosure provides a crystalline form having characteristic peaks at diffraction angles (2θ±0.2°) of 7.0°, 14.0°, 17.5°, 19.5°, 21.0°, 23.7°, and 24.8° in a powder X-ray diffraction spectrum (CuKα).

## Description

### Technical Field

### Cross-Reference to Related Applications

This application claims priority based on Japanese Patent Application No. 2022-019058 filed on February 9, 2022, the entire content of which is incorporated herein by reference. The present disclosure relates to a crystalline form of a cyclohexenone compound useful as a drug substance of a pharmaceutical product, and to a pharmaceutical composition comprising the crystalline form.

### Background Art

Neurodegenerative diseases are broadly divided into central and peripheral diseases according to the site of damaged nerves. Typical diseases of the central nervous system include Alzheimer's disease, amyotrophic lateral sclerosis, and cerebrospinal cord injury. Typical diseases involving peripheral nerve damage include sensory neuropathy such as neuropathic pain and hypoesthesia, and dysautonomia accompanied by constipation or dysuria. Although the pathogenesis of the condition in neurodegenerative diseases varies, the diseases are thought to involve the degeneration or atrophy of neurites and/or neuronal cell death. For those diseases, there is currently a paucity of fundamental therapeutic agents that ameliorate neuropathy.

PTL 1 discloses that 3-(15-hydroxypentadecyl)-2,4,4-trimethylcyclohex-2-en-1-one has a nerve growth stimulation effect and is useful as a medical drug for prevention or treatment of brain diseases such as dementia. PTL 2 discloses that the compound is useful as a therapeutic agent for dysuria. However, 3-(15-hydroxypentadecyl)-2,4,4-trimethylcyclohex-2-en-1-one is unsatisfactory in pharmacokinetics when usually prescribed (PTL 3).

In general, when a compound is used as an active ingredient or a drug substance of a pharmaceutical product, the compound is required to have chemical and physical stability so as to stably maintain the quality and/or to simplify storage management.

### Citation List

### Patent Literature

PTL 1: WO99/08987
PTL 2: WO2002/066024
PTL 3: WO2013/147072

### Summary of Invention

### Technical Problem

An object of the present disclosure is to provide a novel compound with excellent neurite outgrowth action. Another object of the present disclosure is to provide a stable crystalline form of the novel compound useful as an active ingredient or a drug substance of a pharmaceutical product.

### Solution to Problem

The present inventors conducted extensive research to achieve the objects and found that a novel cyclohexenone compound (chemical name: 3-(15-methoxypentadecyl)-2,4,4-trimethylcyclohex-2-en-1-one (sometimes also referred to below as "compound (I)") represented by the following formula (I):

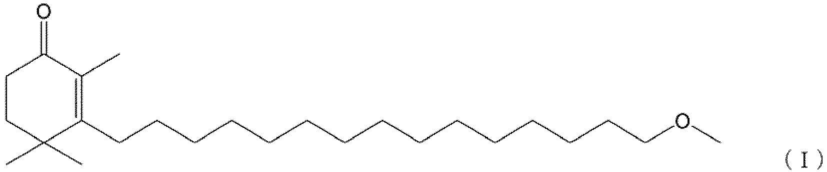

has a nerve growth stimulation effect (neurite outgrowth action and/or action of increasing the proportion of neurite-bearing cells) and is useful as a medical drug for treating diseases that are ameliorated by the promotion of nerve growth (including neurodegenerative diseases), pain, and/or lower urinary tract dysfunction. The present inventors also found that compound (I) has three crystalline forms (type I crystal, type II crystal, and type III crystal), and that these crystalline forms have low moisture absorption and excellent solid stability.

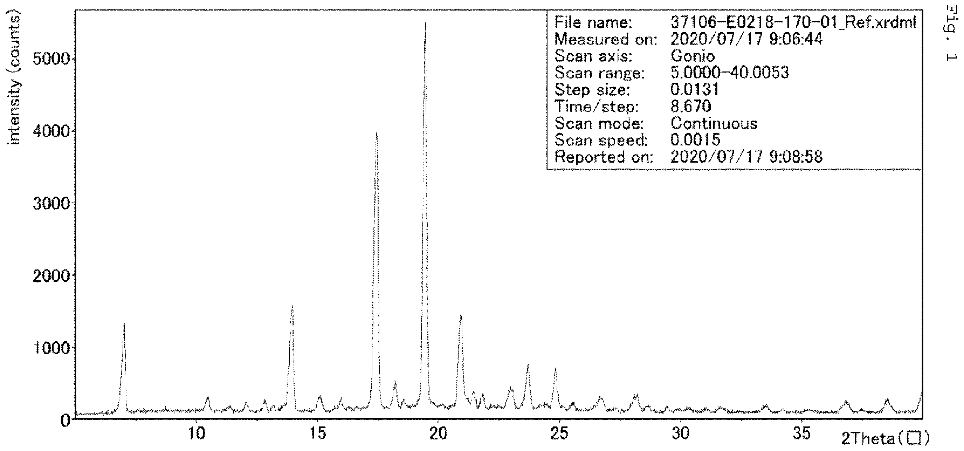

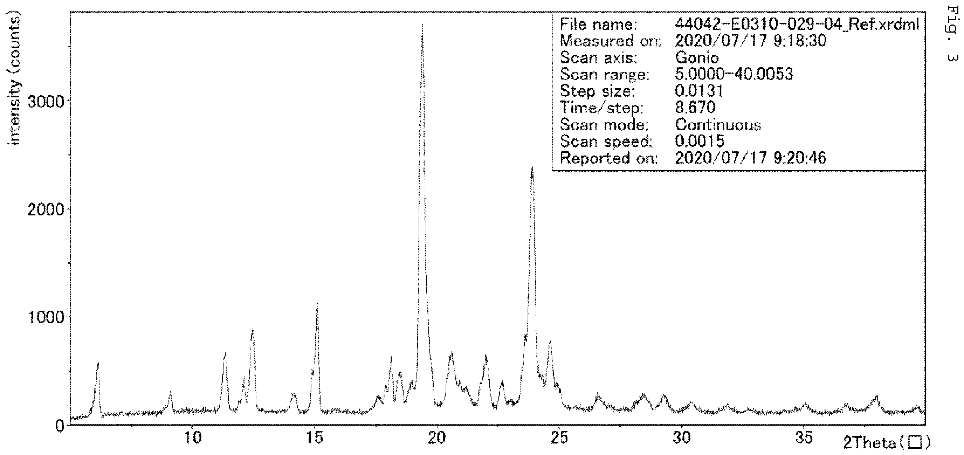

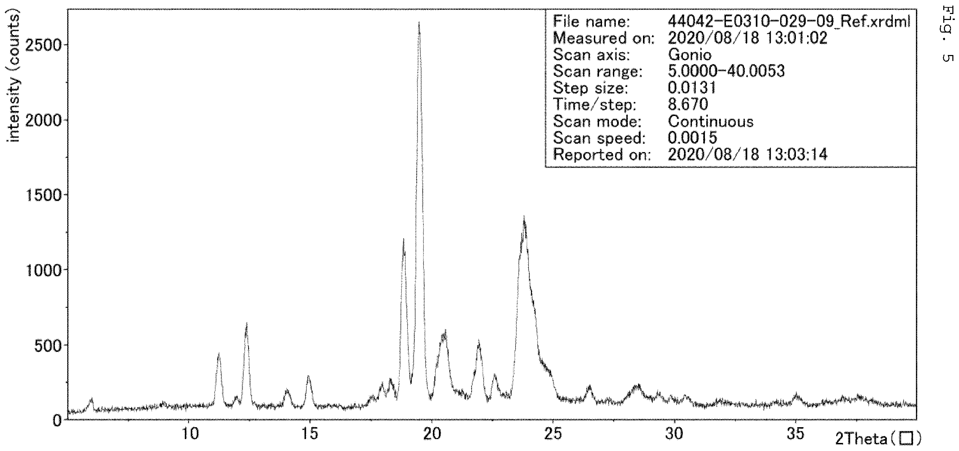

Specifically, the present disclosure provides the following [1] to [15] .
[1] A crystalline form of 3-(15-methoxypentadecyl)-2,4,4-trimethylcyclohex-2-en-1-one having at least three peaks at diffraction angles (2Θ+0.2°) selected from 7.0°, 14.0°, 17.5°, 19.5°, 21.0°, 23.7°, and 24.8° in a powder X-ray diffraction spectrum (CuKα).
[2] The crystalline form according to [1], wherein the crystalline form has peaks at diffraction angles (2Θ+0.2°) of 7.0°, 14.0°, 17.5°, 19.5°, 21.0°, 23.7°, and 24.8° in a powder X-ray diffraction spectrum (CuKα).
[3] The crystalline form according to [1] or [2], wherein the powder X-ray diffraction spectrum is substantially the same as the spectrum shown in Fig. 1.
[4] The crystalline form according to any one of [1] to [3], wherein the crystalline form has an endothermic peak at a peak temperature of around 35°C in differential scanning calorimetry (DSC measurement).
[5] A pharmaceutical composition comprising the crystalline form of any one of [1] to [4].
[6] A crystalline form of 3-(15-methoxypentadecyl)-2,4,4-trimethylcyclohex-2-en-1-one having at least five peaks at diffraction angles (2Θ+0.2°) selected from 6.1°, 11.4°, 12.5°, 15.1°, 19.4°, and 24.0° in a powder X-ray diffraction spectrum (CuKα).
[7] The crystalline form according to [6], wherein the crystalline form has peaks at diffraction angles (2Θ+0.2°) of 6.1°, 11.4°, 12.5°, 15.1°, 19.4°, and 24.0° in a powder X-ray diffraction spectrum (CuKα).
[8] The crystalline form according to [6] or [7], wherein the powder X-ray diffraction spectrum is substantially the same as the spectrum shown in Fig. 3.
[9] The crystalline form according to any one of [6] to [8], wherein the crystalline form has an endothermic peak at a peak temperature (peak-top value) of around 34°C in differential scanning calorimetry (DSC measurement).
[10] A pharmaceutical composition comprising the crystalline form of any one of [6] to [9].
[11] A crystalline form of 3-(15-methoxypentadecyl)-2,4,4-trimethylcyclohex-2-en-1-one having at least five peaks at diffraction angles (2Θ+0.2°) selected from 11.2°, 12.4°, 14.1°, 14.9°, 18.8°, and 19.4° in a powder X-ray diffraction spectrum (CuKα).
[12] The crystalline form according to [11], wherein the crystalline form has peaks at diffraction angles (2Θ+0.2°) of 11.2°, 12.4°, 14.1°, 14.9°, 18.8°, and 19.4° in a powder X-ray diffraction spectrum (CuKα).
[13] The crystalline form according to [11] or [12], wherein the powder X-ray diffraction spectrum is substantially the same as the spectrum shown in Fig. 5.
[14] The crystalline form according to any one of [11] to [13], wherein the crystalline form has an endothermic peak at a peak temperature (peak-top value) of around 34°C in differential scanning calorimetry (DSC measurement).
[15] A pharmaceutical composition comprising the crystalline form of any one of [11] to [14].

### Advantageous Effects of Invention

The present disclosure provides compound (I) described above, which is useful as a compound with a nerve growth stimulation effect. The present disclosure also provides compound (I) described above, which is useful as a compound with excellent pharmacokinetics and/or action of improving urinary function. Compound (I) of the present disclosure is a novel compound. Its crystalline forms, the stable crystalline forms of compound (I), and their production methods are currently unknown. In the present disclosure, the crystalline forms (the type I crystal, type II crystal, and type III crystal) of compound (I) are superior to other crystalline forms in terms of, for example, handling properties (lower moisture absorption) and/or quality control, and are thus useful when the compound is used as a drug substance of a pharmaceutical product.

Additionally, the type I crystal, type II crystal, and type III crystal of the present disclosure are safe as a pharmaceutical product since the residual solvents are below the standard values specified in the guideline on residual solvents of pharmaceutical products in the ICH guidelines.

### Brief Description of Drawings

Fig. 1: A powder X-ray diffraction spectrum (CuKα) of the type I crystal of compound (I) obtained in Example 1 (the vertical axis represents intensity (counts), and the horizontal axis represents diffraction angle (2θ)).
Fig. 2: A differential scanning calorimetry (DSC measurement) curve of the type I crystal of compound (I) obtained in Example 1.
Fig. 3: A powder X-ray diffraction spectrum (CuKα) of the type II crystal of compound (I) obtained in Example 2 (the vertical axis represents intensity (counts), and the horizontal axis represents diffraction angle (2θ)).
Fig. 4: A differential scanning calorimetry (DSC measurement) curve of the type II crystal of compound (I) obtained in Example 2.
Fig. 5: A powder X-ray diffraction spectrum (CuKα) of the type III crystal of compound (I) obtained in Example 3 (the vertical axis represents intensity (counts), and the horizontal axis represents diffraction angle (2θ)).
Fig. 6: A differential scanning calorimetry (DSC measurement) curve of the type III crystal of compound (I) obtained in Example 3.
Fig. 7: A moisture adsorption/desorption isotherm of the type I crystal of compound (I) obtained in Example 1.
Fig. 8: A moisture adsorption/desorption isotherm of the type II crystal of compound (I) obtained in Example 2.
Fig. 9: A moisture adsorption/desorption isotherm of the type III crystal of compound (I) obtained in Example 3.
Fig. 10: A graph of residual urine volume of each group. The values represent mean + S.E. The number n of each group is 10, 10, 12, and 12 from left to right.

*: p<0.05, there is a significant difference compared with the vehicle in Dunnett's test
NS: there is no significant difference compared with the vehicle in Dunnett's test
#: p<0.05, there is a significant difference compared with compound (I) in Student's t-test
##: p<0.01, there is a significant difference compared with the vehicle in Student's *t*-test

### Description of Embodiments

In the present specification, the singular forms (e.g., a, an, and the) shall include both the singular and plural forms, unless otherwise specified herein or clearly contradicted by context. Unless otherwise specified, the phrase "compound (I)" when simply mentioned in the present specification refers to "3-(15-methoxypentadecyl)-2,4,4-trimethylcyclohex-2-en-1-one" and is used to mean both "amorphous form" and "crystal."

Compound (I) can be synthesized, for example, according to the method described in Synthesis Example 1 below; however, the method is not limited to this method.

In the present specification, the terms "crystal" and "amorphous form" are used with their ordinary meanings in the technical field of pharmaceuticals to which the present disclosure belongs. In the present specification, the term "crystal" and related terms used in the present specification, such as "crystalline form" and "crystalline morphology," are used interchangeably. Further, the term "amorphous" and related terms used in the present specification, such as "amorphous form" and "amorphous morphology," are used interchangeably.

The crystals of the present disclosure can be isolated and purified by known separation and purification techniques, such as recrystallization, crystallization, distillation, and column chromatography.

As used herein and unless otherwise specified, the term "crystal" and related terms used in the present specification to refer to a compound, substance, modification, material, component, or product, indicate that the compound, substance, modification, material, component, or product is substantially crystalline as determined by X-ray diffraction. See, for example, Remington: The Science and Practice of Pharmacy, 21st edition, Lippincott, Williams and Wilkins, Baltimore, MD (2005); and The US Pharmacopeia, 23rd edition, 1843-1844 (1995).

As used herein and unless otherwise specified, the term "crystalline morphology" and related terms in the present specification refer to a solid form that is crystalline. The crystalline morphology includes single-component crystalline morphology and multi-component crystalline morphology, which may optionally, but not by way of limitation, include co-crystals, salts (including pharmaceutically acceptable salts), polymorphic forms, solvates, hydrates, and/or other molecular complexes. In a specific embodiment, it is possible that the crystalline morphology of a substance does not substantially contain an amorphous morphology and/or other crystalline morphologies. In a specific embodiment, the crystalline morphology of a substance may contain less than about 0.1, 0.5, 1, 2, 3, 4, 5, 6, 7, 8, 9, 10, 11, 12, 13, 14, 15, 20, 25, 30, 35, 40, 45, or 50% of one or more amorphous morphology and/or other crystalline morphologies on a weight basis.

The term "co-crystal" refers to a molecular complex derived from many co-crystal-forming substances known in this technical field. Unlike salts, co-crystals typically do not involve hydrogen ion transfer between co-crystal-forming substances and drugs. Instead, they involve intermolecular interactions, such as hydrogen bonding, aromatic-ring stacking, or dispersion forces between co-crystal-forming substances and compound (I) in the crystal structure.

As used herein and unless otherwise specified, the terms "polymorphic form" and "polymorphic morphology," and related terms in the present specification refer to two or more crystalline morphologies composed of substantially the same molecules, molecular groups, and/or ions. Like different crystalline morphologies, different polymorphic forms also can have, for example, different physical properties, such as melting point, enthalpy of fusion, solubility, dissolution characteristics, and/or vibrational spectra (as a result of the arrangement or conformation of molecules and/or ions in the crystal lattice). The differences in physical properties may affect pharmaceutical parameters, such as storage stability, compressibility and density (which are important in formulation and production of a product), and dissolution rate (which is an important factor in bioavailability). Differences in stability can be caused by changes due to chemical reactivity (for example, oxidation difference such that a dosage form composed of one polymorphic form undergoes a change in color faster than a dosage form composed of another polymorphic form) or mechanical changes (for example, a tablet will disintegrate during storage when a kinetically favorable polymorphic form is transformed into a thermodynamically more stable polymorphic form) or both (for example, some tablet of a polymorphic form is more easily degraded at high humidity). As a result of differences in solubility/dissolution properties, solid transition can partly cause loss of efficacy in some extreme cases and can cause toxicity in other extreme cases. In addition, physical properties can be important in processing (for example, some polymorphic forms may be relatively prone to solvate formation, or may not be easily filtered or washed to remove impurities; additionally, the particle shape and particle size distribution may vary among polymorphic forms).

As used herein and unless otherwise specified, the terms "amorphous" and "amorphous morphology," and related terms used in the present specification indicate that the substance, component, or product is not substantially crystalline as determined by X-ray diffraction. In particular, the term "amorphous morphology" describes an irregular solid form, i.e., a solid form lacking long-range crystalline order.

The term "pharmaceutically acceptable salt" refers to a salt derived from various organic and inorganic counterions known in this technical field. The pharmaceutically acceptable salts can be safe for ingestion by animals or humans.

Techniques for characterizing crystalline and amorphous morphologies include, but are not limited to, thermogravimetric analysis (TGA), differential scanning calorimetry (DSC), X-ray powder diffraction (XRPD), single-crystal X-ray diffraction, vibrational spectroscopy such as infrared (IR) and Raman spectroscopy, solid-state and solution nuclear magnetic resonance (NMR) spectroscopy, optical microscopic observation, hot stage optical microscopic observation, scanning electron microscopic observation (SEM), electron crystallography and quantitative analysis, particle size analysis (PSA), surface area analysis, solubility measurements, dissolution measurements, atomic analysis, and Karl Fischer analysis. Characteristic unit cell parameters can be determined by using one or more techniques such as, but not limited to, X-ray diffraction and neutron diffraction, including single crystal diffraction and powder diffraction. Techniques useful for analyzing powder diffraction data include profile refinement, such as Rietveld refinement, which can be used, for example, to analyze diffraction peaks associated with a single phase in a sample containing two or more solid phases. Other methods useful for analyzing powder diffraction data include unit cell indexing, which allows a person skilled in the art to determine unit cell parameters from a sample containing crystalline powder.

In powder X-ray diffraction patterns, due to the nature of the data, diffraction angles and overall patterns may be important when recognizing the crystal identity. The relative intensity in a powder X-ray diffraction pattern may vary slightly depending on the direction of crystal growth, particle size, measurement conditions, and the like, and thus should not be strictly interpreted.

The numerical values obtained from various patterns may have slight errors depending on the direction of crystal growth, particle size, measurement conditions, and the like. Therefore, in this specification, the numerical values of diffraction angles (2θ) in a powder X-ray diffraction pattern may have a measurement error within the range of approximately ±0.2°.

For an endothermic peak in a differential scanning calorimetry (DSC measurement) curve, the measurement temperature may vary depending on the rate of temperature increase per minute, sample purity, and the like. In the present specification, the term "around" for measurement temperature values in DSC means that the temperature value is within ±2°C, ±3°C, ±4°C, or +5°C of that value.

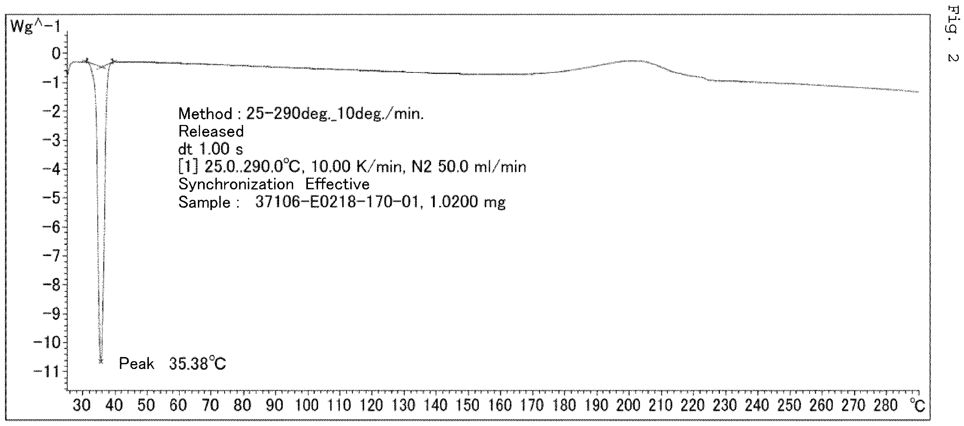

In one embodiment of the present disclosure, the type I crystal of compound (I) exhibits, for example, the powder X-ray diffraction spectrum (CuKα) shown in Fig. 1 and also exhibits the differential scanning calorimetry (DSC measurement) curve shown in Fig. 2.

In the powder X-ray diffraction spectrum (CuKα) of the type I crystal of compound (I), characteristic peaks may be observed at diffraction angles (2Θ+0.2°) of 7.0°, 14.0°, 17.5°, 19.5°, 21.0°, 23.7°, and 24.8°.

The type I crystal of compound (I) of the present disclosure is a crystalline form having at least three peaks selected from the above peaks, preferably having at least five peaks selected from the above peaks, and particularly preferably having all of the above peaks.

In the differential scanning calorimetry (DSC measurement) curve of the type I crystal of compound (I), an endothermic peak may be observed, for example, at around 33°C to 37°C, preferably around 34°C to 36°C, and more preferably around 35°C.

The crystalline morphology according to the present disclosure may be characterized by a combination of the embodiments described above, which do not contradict each other. For example, a crystalline morphology may be characterized by a combination of the peaks in an X-ray diffraction pattern and the endothermic peak as measured by DSC.

In one embodiment, the crystalline morphology disclosed in the present specification can include multiple crystals with spatially regular arrangements of atoms (a crystal polymorphic form) and can give different physicochemical properties. In one embodiment, the crystalline morphology disclosed in the present specification may be a mixture comprising a crystal polymorphic form.

In one embodiment, the crystalline morphology of compound (I) disclosed in the present specification may be physically and/or chemically pure. In a specific embodiment, the crystalline morphology of the compound disclosed in the present specification can have physical and/or chemical purity of at least about 100, 99, 98, 97, 96, 95, 94, 93, 92, 91, 90, 89, 88, 87, 86, 85, 84, 83, 82, 81, or 80%. In one embodiment, the crystalline morphology of the compound disclosed is the present specification is substantially pure. As used herein and unless otherwise specified, a sample that is "substantially pure," for example, a sample that contains a specific crystalline morphology or amorphous morphology and that does not substantially contain other solid forms and/or other compounds contains, in a specific embodiment, about less than 25%, 20%, 15%, 10%, 9%, 8%, 7%, 6%, 5%, 4%, 3%, 2%, 1%, 0.75%, 0.5%, 0.25%, or 0.1% by weight of one or more other solid forms and/or other compounds.

In one embodiment, the crystalline morphology of compound (I) disclosed in the present specification is stable upon exposure to the conditions of about 25°C, 30°C, or 35°C and about 65, 70, 75, 80, or 85% relative humidity for about 1, 2, 3, 4, 5, 6, 7, 8, 12, 16, 24, 36, 48, 60, 72, 84, 96, 108, 120, 132, 144, or 156 weeks or more and about 160, 156, 150, 138, 126, 114, 102, 90, 78, 66, 54, 42, 30, 24, 18, 12, or 6 weeks or less. The conditions can be closed or open conditions. As used herein, the "closed" conditions can mean that the lid of a bottle containing the sample is closed or hermetically sealed during the stability experiment, while the "open" conditions can mean that the lid is open. In one embodiment, the crystalline morphology of compound (I) disclosed in the present specification is stable upon exposure to the conditions of about 25°C and about 60% relative humidity for about 4 weeks. In one embodiment, the conditions are closed conditions. That is, the crystalline morphology of the compound disclosed in the present specification has excellent storage stability over a long period of time. In the present specification, as being "stable" means that the increase in the amount of impurities is no more than about 1.0, 0.5, 0.3, 0.1, 0.05, or 0.01%, compared with the original amount of impurities. The stability can also be demonstrated, for example, by maintaining 10, 20, 30, 40, 50, 60, 70, 80, or 90% or more of the original peaks at (2θ + 0.2°) in an X-ray diffraction pattern.

The crystalline morphology of the present disclosure may be a solvate (e.g., a hydrate) or a non-solvate.

Compounds labeled with isotopes (e.g., deuterium, 3H, 14C, 35S, and 125I) and the like are also included in the compound of the present specification or a pharmaceutically acceptable salt thereof.

In one embodiment of the present disclosure, the type I crystal of compound (I) can be obtained by, for example, a method comprising
step (i-1) of adding compound (I) to solvent I-1 for dissolution, and
step (i-2) of adding solvent I-2 to the solution of compound (I) obtained in step (i-1), followed by stirring to crystallize compound (I). In this embodiment, examples of solvent I-1 include methanol, ethanol, 1-propanol, 2-propanol, and acetone.

In step (i-1), the amount of compound (I) added is not particularly limited. For example, compound (I) can be added in an amount of 0.1 to 20 g and preferably 0.5 to 5 g, on a mass basis, per 10 mL of solvent I-1. The temperature in step (i-1) is appropriately set according to the solvent used and is set between 20°C and the boiling point of the solvent.

In the above embodiment, solvent I-2 may be, for example, water. The ratio of solvent I-1 and solvent I-2 (v/v) can be, for example, such that 0 to 50 ml and preferably 0 to 20 ml of solvent I-2 is used per 10 ml of solvent I-1. The temperature in the crystallization in step (i-2) is appropriately set according to the solvent used and is set between -20°C and 30°C.

Compound (I) can also be crystallized by adding solvent I-1 and solvent I-2 simultaneously and heating the mixture for dissolution, followed by cooling with stirring.

The stirring in the crystallization in step (i-2) is performed by using a stirrer, stirring blades, a magnetic stirrer, etc. as appropriate according to, for example, the amounts of the solvents and the size of the reaction vessel. The stirring speed is usually 1 to 1200 rpm, and preferably 10 to 600 rpm.

The type I crystal obtained as described above has a chemical purity of compound (I) of 90% or more and can be measured by high-performance liquid chromatography (HPLC). The type I crystal preferably has a chemical purity of compound (I) of 95% or more, and more preferably 99% or more.

The type I crystal of compound (I), which comprises the type I crystal, may be a single crystal of the type I crystal or may be a polymorphic mixture also comprising other crystals. The type I crystal of compound (I) preferably comprises 90 wt% or more and more preferably 95 wt% or more of the type I crystal.

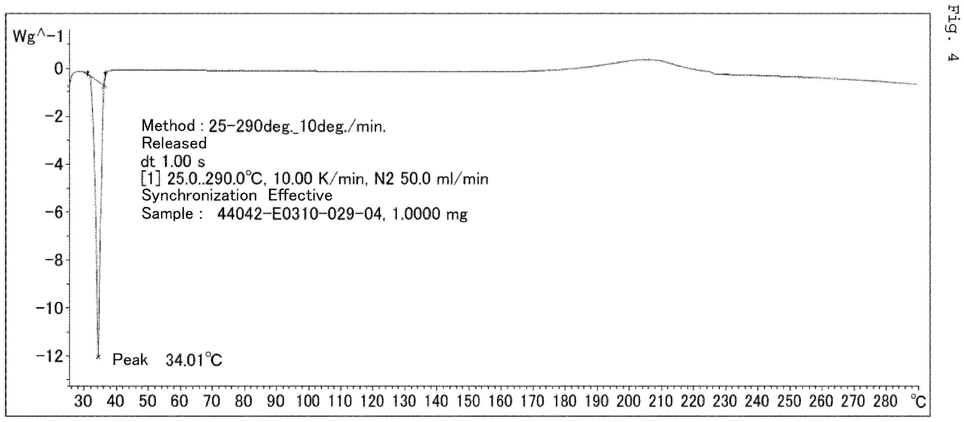

In one embodiment of the present disclosure, the type II crystal of compound (I) exhibits, for example, the powder X-ray diffraction spectrum (CuKα) shown in Fig. 3 and also exhibits the differential scanning calorimetry (DSC measurement) curve shown in Fig. 4.

In the powder X-ray diffraction spectrum (CuKα) of the type II crystal of compound (I), characteristic peaks may be observed at diffraction angles (2Θ+0.2°) of 6.1°, 11.4°, 12.5°, 15.1°, 19.4°, and 24.0°.

The type II crystal of compound (I) of the present disclosure is a crystal having at least five peaks selected from the above peaks, and particularly preferably having all of the above peaks.

In the differential scanning calorimetry (DSC measurement) curve of the type II crystal of compound (I), an endothermic peak may be observed, for example, at around 32°C to 36°C, preferably around 33°C to 35°C, and more preferably around 34°C.

In one embodiment of the present disclosure, the type II crystal of compound (I) can be obtained by, for example, a method comprising
step (ii-1) of adding compound (I) to solvent II for dissolution, and
step (ii-2) of cooling the solution of compound (I) obtained in
step (ii-1) to crystallize compound (I).
In this embodiment, solvent II may be, for example, acetonitrile.

In step (ii-1), the amount of compound (I) added is not particularly limited. For example, compound (I) can be added in an amount of 1 to 4 g and preferably 1.5 to 3.5 g, on a mass basis, per 10 mL of solvent II. The temperature in step (ii-1) is set appropriately and is set between 15°C and 40°C. The temperature in the crystallization in step (ii-2) is set between -10°C to 5°C. It is preferable that the temperature is cooled from the temperature in step (ii-1) to a temperature lower than 3°C and then maintained at a temperature lower than 3°C.

The crystallization in step (ii-2) may be performed with stirring. In this case, the stirring is performed by using a stirrer, stirring blades, etc. as appropriate according to, for example, the amount of the solvent and the size of the reaction vessel. The stirring operation can also be performed, for example, by shaking the reaction vessel. The stirring speed is usually 1 to 600 rpm, and preferably 10 to 100 rpm.

The type II crystal obtained as described above has 90% or more chemical purity of compound (I) and can be measured by high-performance liquid chromatography (HPLC). The type II crystal preferably has a chemical purity of compound (I) of 95% or more, and more preferably 99% or more.

The type II crystal of compound (I), which comprises the type II crystal, may be a single crystal of the type II crystal or may be a polymorphic mixture also comprising other crystals. The type II crystal of compound (I) preferably comprises 90 wt% or more and more preferably 95 wt% or more of the type II crystal.

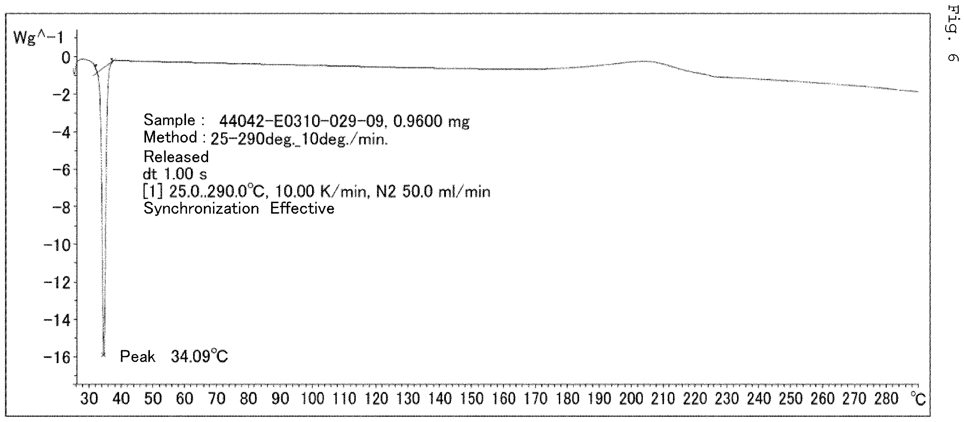

In one embodiment of the present disclosure, the type III crystal of compound (I) exhibits, for example, the powder X-ray diffraction spectrum (CuKα) shown in Fig. 5 and also exhibits the differential scanning calorimetry (DSC measurement) curve shown in Fig. 6.

In the powder X-ray diffraction spectrum (CuKα) of the type III crystal of compound (I), characteristic peaks may be observed at diffraction angles (2Θ+0.2°) of 11.2°, 12.4°, 14.1°, 14.9°, 18.8°, and 19.4°.

The type III crystal of compound (I) of the present disclosure is a crystal having at least five peaks selected from the above peaks, and particularly preferably having all of the above peaks.

In the differential scanning calorimetry (DSC measurement) curve of the type III crystal of compound (I), an endothermic peak may be observed, for example, at around 32°C to 36°C, preferably around 33°C to 35°C, and more preferably around 34°C.

In one embodiment of the present disclosure, the type III crystal of compound (I) can be obtained by, for example, a method comprising
step (iii-1) of adding the type II crystal of compound (I) to
solvent III-1 and stirring the mixture, and
step (iii-2) of recovering the solid of compound (I) from the suspension of compound (I) obtained in step (iii-1) above.
In this embodiment, solvent III-1 may be a mixed solvent of methanol and water. When a mixed solvent of methanol and water is used, the ratio (v/v) can be, for example, such that 1 to 15 ml and preferably 5 to 11 ml of water is used per 100 ml of methanol.

In step (iii-1), the amount of the type II crystal of compound (I) added is not particularly limited. For example, compound (I) can be added in an amount of 1 to 5 g and preferably 1.5 to 2.5 g, on a mass basis, per 10 mL of solvent III-1. The temperature in step (iii-1) is appropriately set according to the solvent used and is set between 0°C and 8°C.

The recovery method used in step (iii-2) may be selected from various methods that can be used in the technical field to which the present disclosure belongs, such as filtration, washing with an organic solvent, and vacuum drying.

The stirring in step (iii-1) is performed by using a stirrer, stirring blades, a magnetic stirrer, etc. as appropriate according to, for example, the amount of the solvent and the size of the reaction vessel. The stirring speed is usually 1 to 600 rpm, and preferably 10 to 300 rpm.

The type III crystal obtained as described above has 90% or more chemical purity of compound (I) and can be measured by high-performance liquid chromatography (HPLC). The type III crystal preferably has a chemical purity of compound (I) of 95% or more, and more preferably 99% or more.

The type III crystal of compound (I), which comprises the type III crystal, may be a single crystal of the type III crystal or may be a polymorphic mixture also comprising other crystals. The type III crystal of compound (I) preferably comprises 90 wt% or more, and more preferably 95 wt% or more of the type III crystal.

As demonstrated in the Examples below, the type I crystal, type II crystal, and type III crystal of compound (I) according to the present disclosure all have low moisture absorption and excellent solid stability (e.g., storage stability). It is important for a drug substance of a pharmaceutical product and an active ingredient contained in a pharmaceutical product to have solid stability both in industrial operations and in maintaining quality.

Solvents used in the production of pharmaceutical products can be toxic, and in terms of safety, it is desirable that as little solvent as possible remains in the production process. In this respect, the type I crystal, type II crystal, and type III crystal of compound (I) do not contain residual solvents beyond the regulatory limit of the ICH (International Council for Harmonisation of Technical Requirements for Pharmaceuticals for Human Use) guideline.

Therefore, the type I crystal, type II crystal, and type III crystal of compound (I) according to the present disclosure have excellent properties required as a pharmaceutical product or a drug substance of a pharmaceutical product.

The type I crystal, type II crystal, and type III crystal of compound (I) according to the present disclosure have excellent neurite outgrowth action. For example, due to neurite outgrowth action and/or action of increasing the proportion of neurite-bearing cells, the type I crystal, type II crystal, and type III crystal of compound (I) according to the present disclosure are useful as a pharmaceutical product for the treatment of diseases that are ameliorated by the promotion of nerve growth (including neurodegenerative diseases), pain, and/or lower urinary tract dysfunction.

For use of the type I crystal, type II crystal, and/or type III crystal of compound (I) as a medical drug, various dosage forms can be used according to preventive or treatment purposes, with or without pulverization of the type I crystal, type II crystal, and/or type III crystal. Examples of the form include oral drugs, such as tablets, capsules, granules, fine granules, powders, and dry syrups; and non-oral drugs, such as suppositories, inhalants, nasal drops, ointments, patches, and injections. The form of an oral drug is preferred. These pharmaceutical compositions can be produced according to formulation methods that would be known and commonly used by a person skilled in the art by using a pharmaceutically acceptable carrier.

The pharmaceutical carrier for use can be various organic or inorganic carrier substances commonly used as a formulation material. The pharmaceutical carrier is added, for example, as an excipient, a binder, a disintegrant, a lubricant, or a coating agent in a solid formulation, or as a solvent, a solubilization agent, a suspending agent, a tonicity agent, a buffer, or a soothing agent in a liquid formulation. Additionally, formulation additives, such as a preservative, an antioxidant, a colorant, a sweetener, and a stabilizer, may optionally be used.

Examples of excipients include lactose, sucrose, D-mannitol, starch, crystalline cellulose, and calcium silicate.

Examples of binders include hydroxypropyl cellulose, methyl cellulose, polyvinylpyrrolidone, candy powder, and hypromellose.

Examples of disintegrants include sodium starch glycolate, carmellose calcium, croscarmellose sodium, crospovidone, low-substituted hydroxypropyl cellulose, and partially pregelatinized starch.

Examples of lubricants include talc, magnesium stearate, sucrose fatty acid ester, stearic acid, and sodium stearyl fumarate.

Examples of coating agents include ethyl cellulose, aminoalkyl methacrylate copolymer RS, hypromellose, and sucrose.

Examples of solvents include water, propylene glycol, and a saline solution.

Examples of solubilization agents include polyethylene glycol, ethanol, α-cyclodextrin, macrogol 400, and polysorbate 80.

Examples of suspending agents include carrageenan, crystalline cellulose-carmellose sodium, and polyoxyethylene hydrogenated castor oil.

Examples of tonicity agents include sodium chloride, glycerin, and potassium chloride.

Examples of pH adjusters and buffers include sodium citrate, hydrochloric acid, lactic acid, phosphoric acid, and sodium dihydrogen phosphate.

Examples of soothing agents include procaine hydrochloride and lidocaine.

Examples of preservatives include ethyl paraoxybenzoate, cresol, and benzalkonium chloride.

Examples of antioxidants include sodium sulfite, ascorbic acid, and natural vitamin E.

Examples of colorants include titanium oxide, iron sesquioxide, Food Blue No. 1, and copper chlorophyll.

Examples of taste-masking or odor-masking agents include aspartame, saccharin, sucralose, l-menthol, and mint flavor.

Examples of stabilizers include sodium pyrosulfite, sodium edetate, erythorbic acid, magnesium oxide, and dibutylhydroxytoluene.

Oral solid preparations can be prepared by adding an excipient, and optionally a binder, disintegrant, lubricant, colorant, taste-masking or odor-masking agent, etc. to the type I crystal, type II crystal, and/or type III crystal of compound (I), and formulating the resulting mixture into tablets, coated tablets, granules, powders, capsules, or the like by ordinary methods.

The amount of the type I crystal, type II crystal, and/or type III crystal of compound (I) to be contained in each unit dosage form is preferably about 0.05 to 1000 mg for an oral drug, about 0.1 to 500 mg for an injection, and about 1 to 1000 mg for a suppository or external drug, typically per unit dosage form, although the amount is not constant, for example, due to the symptoms of the patient to be treated or the dosage form.

Although the daily dose of the drug of the type I crystal, type II crystal, and/or type III crystal of compound (I) in the dosage forms described above cannot be generalized due to the variation of the patient's symptoms, body weight, age, gender, etc., the daily dose may be usually about 0.05 to 5000 mg, preferably 0.1 to 1000 mg, for an adult (body weight: 50 kg). The drug in this dosage can be administered once daily or in about two to three divided doses daily.

### Examples

The present disclosure is described in more detail below with reference to Examples. However, the present disclosure is not limited to the Examples. Although the present disclosure is sufficiently described by the Examples, a person skilled in the art would understand that various changes and/or modifications are available. Such changes and/or modifications are thus included in the present disclosure unless they depart from the spirit and principal concept of the present disclosure.

The reagents used in the Examples are commercially available products unless indicated otherwise. NMR spectra were obtained using an AL400 NMR Spectrometer (400 MHz; produced by JEOL). Tetramethylsilane was used as the internal reference when the deuterated solvent contained tetramethylsilane.
Otherwise, the measurement was performed using the peak of residual non-deuterated proton in the NMR solvent as the internal standard. The δ values are all shown in ppm.

The symbols stand for the following.
s: Singlet
t: Triplet
m: Multiplet
CDCl₃: Deuterated chloroform

### Powder X-ray Diffraction Measurement

Powder X-ray diffraction was measured under any of the following test conditions after lightly pulverizing an appropriate amount of a test material in an agate mortar as necessary.

Apparatus: Empyrean, produced by PANalytical Reflective method (focusing method)
Target: Cu
X-ray tube current: 40 mA
X-ray tube voltage: 45 kV
Scanning range: 2θ = 5.0 to 40.0°
Step: 2θ = 0.0131°
Average time/step: 8.670 s
Scanning speed: 0.0015°/s
Divergence slit: 1°
Scattering slit: 2.0 mm
Light-receiving slit: 8.0 mm

The handling of the apparatus, including data processing, was in accordance with the method and procedure as instructed for each apparatus.

The numerical values obtained from various spectra may vary slightly depending on the direction of crystal growth, particle size, measurement conditions, and the like, and thus should not be strictly interpreted.

### Differential Scanning Calorimetry (DSC Measurement)

DSC measurement was performed according to the following test conditions.

Apparatus: DSC1 STAR System, produced by Mettler Toledo
Sample: about 1 mg
Sample container: made of aluminum
Temperature increase range: 25 to 290°C
Temperature increase rate: 10°C/min
Atmospheric gas: Nitrogen
Nitrogen gas flow: 50 mL/min
The handling of the apparatus, including data processing, was in accordance with the method and procedure as instructed for each apparatus.

3-(15-Hydroxypentadecyl)-2,4,4-trimethylcyclohex-2-en-1-one described below may also be referred to as "compound A."

### Synthesis Example 1: Synthesis of 3-(15-methoxypentadecyl)-2,4,4-trimethylcyclohex-2-en-1-one (Compound (I))

A solution of 72.9 g of 3-(15-hydroxypentadecyl)-2,4,4-trimethylcyclohex-2-en-1-one in 150 mL of tetrahydrofuran was added dropwise at ice cooling temperature to a solution of 48.1 g of sodium tert-butoxide in 550 mL of tetrahydrofuran, and the mixture was stirred for 30 minutes. Then, a solution of 56.8 g of iodomethane in 50 mL of tetrahydrofuran was added to the reaction mixture and stirred at ice cooling temperature for 0.5 hours and at room temperature for 1.5 hours. At ice cooling temperature, a saturated ammonium chloride solution was added to the reaction mixture, and the resulting mixture was extracted with ethyl acetate, washed with water and brine, and dried over anhydrous sodium sulfate. After the desiccant was filtered off, the residue obtained after evaporation under reduced pressure was purified by silica gel column chromatography (ethyl acetate/hexane) to give 61.5 g of the title compound.

### Example 1: Production of Type I Crystal of Compound (I)

Methanol (4 mL) and water (3 mL) were added to 1.00 g of compound (I), and the mixture was heated and stirred. The mixture was then cooled to room temperature with stirring. The precipitate was collected by filtration and dried under reduced pressure at room temperature to give 892 mg of the title crystal. The ¹H-NMR spectrum of the obtained compound was as follows.
NMR (CDCl3) δppm 1.15 (s, 6H), 1.20-1.48 (m, 24H), 1.50-1.63 (m, 2H), 1.75 (s, 3H), 1.75-1.85 (m, 2H), 2.13-2.24 (m, 2H), 2.45 (t, J = 6.8 Hz, 2H), 3.33 (s, 3H), 3.36 (t, J = 6.8 Hz, 2H).

Fig. 1 shows the powder X-ray diffraction spectrum (CuKα).

The characteristic diffraction angles were as follows. Characteristic diffraction angles (2θ+0.2°):
7.0°, 14.0°, 17.5°, 19.5°, 21.0°, 23.7°, and 24.8°(CuKcx)

The results of the differential scanning calorimetry (DSC) of the type I crystal are as follows.
Differential scanning calorimetry curve: shown in Fig. 2 Endothermic peak (peak-top value) in differential scanning calorimetry curve: around 35°C

### Example 2: Production of Type II Crystal of Compound (I)

Acetonitrile (4 mL) was added to 1000 mg of compound (I) for dissolution. The resulting product was cooled on ice, the precipitate was collected by filtration, and the solid was recovered and dried under reduced pressure at room temperature for about 45 minutes to give 747.0 mg of the title crystal.

Fig. 3 shows the powder X-ray diffraction spectrum (CuKα).

The characteristic diffraction angles were as follows. Characteristic diffraction angles (2θ+0.2°):
6.1°, 11.4°, 12.5°, 15.1°, 19.4°, and 24.0° (CuKcx)

The results of the differential scanning calorimetry (DSC) of the type II crystal are as follows.
Differential scanning calorimetry curve: shown in Fig. 4 Endothermic peak (peak-top value) in differential scanning calorimetry curve: around 34°C

### Example 3: Production of Type III Crystal of Compound (I)

An amount of 3.25 mL of a liquid mixture of methanol/water (9:1 v/v) was added to 650 mg of the type II crystal of compound (I) obtained in Example 2, and the suspension was stirred at 4°C for about 100 minutes. Subsequently, filtration was performed, and the solid was recovered and dried under reduced pressure at room temperature for about 25 minutes. An amount of 2.5 mL of a liquid mixture of methanol/water (9:1 v/v) was added to 500 mg of the solid, and the suspension was stirred at 4°C for about 19 hours. Subsequently, filtration was performed, and the solid was recovered and dried under reduced pressure at room temperature for about 25 minutes to give 335.6 mg of the title crystal.

Fig. 5 shows the powder X-ray diffraction spectrum (CuKα).

The characteristic diffraction angles were as follows. Characteristic diffraction angles (2θ+0.2°):
11.2°, 12.4°, 14.1°, 14.9°, 18.8°, and 19.4°

The results of the differential scanning calorimetry (DSC) of the type III crystal are as follows.
Differential scanning calorimetry curve: shown in Fig. 6 Endothermic peak (peak-top value) in differential scanning calorimetry curve: around 34°C

### Test Example 1: Solid Stability Test

The solid stability of the type I crystal, type II crystal, and type III crystal of compound (I) obtained in Examples 1 to 3 was evaluated after storage for 4 weeks.
Storage condition: 25°C/60% RH (closed system)
Storage period: 4 weeks
Storage amount: about 25 mg
Storage container: glass bottle

Changes in the purity of compound (I) and in the amounts of related substances (the amounts of substances detected other than compound (I)) were analyzed by weighing about 1 mg of the sample, dissolving it in 2 mL of acetonitrile, and accurately measuring 5 µL of this liquid for analysis by HPLC according to the following method. The handling of the apparatus, including data processing, was in accordance with the method and procedure as instructed for each apparatus.

Column: YMC-Triart C8 (4.6 × 150 mm, 3 µm), produced by YMC Co., Ltd.
UV detection: 248 nm
Column temperature: 40°C
Flow rate: 1 mL/min
Sample cooler: 25°C
Sample concentration: 0.5 mg/mL
Mobile phase A: Liquid mixture of water/acetonitrile (1:1 v/v)
Mobile phase B: Acetonitrile

The gradients are shown in Table 1.

**Table 1**

| Time (min) | Mobile phase A (vol%) | Mobile phase B (vol%) |
|---|---|---|
| 0 | 47 | 53 |
| 16 | 0 | 100 |
| 30 | 0 | 100 |
| 31 | 47 | 53 |
| 40 | 47 | 53 |

The results are shown in Table 2 below.

**Table 2**

| | **Before storage** | **After 4 weeks of storage under sealed conditions at 25°C** | **Change in purity** |
|---|---|---|---|
| Type I crystal | 99.70% | 99.71% | +0.01% |
| Type II crystal | 99.73% | 99.72% | -0.01% |
| Type III crystal | 99.77% | 99.77% | - |

As shown in Table 2, the type I crystal, type II crystal, and type III crystal of compound (I) all showed almost no change in purity. Additionally, the type I crystal, type II crystal, and type III crystal of compound (I) all showed almost no increase in related substances. These results indicate that the type I crystal, type II crystal, and type III crystal of compound (I) are all extremely stable crystals.

### Test Example 2: Dynamic Moisture Adsorption/Desorption Test

The type I crystal, type II crystal, and type III crystal of compound (I) obtained in Examples 1 to 3 were subjected to a moisture adsorption/desorption test after storage for 4 weeks.

The moisture adsorption/desorption test was performed according to the following conditions.

About 10 mg of the sample was filled in a dedicated quartz holder, and the weight of the sample at each humidity was continuously measured and recorded under the following conditions. The handling of the apparatus, including data processing, was in accordance with the method and procedure as instructed for each apparatus.
Apparatus: VTI-SA+ (TA Instruments, Inc.)
Drying temperature: 25°C
Temperature increase rate: 1°C/min
Equilibrium of drying: No decrease by 0.01 wt% in 5 minutes was confirmed within the range that did not exceed 120 minutes.
Measurement temperature: 25°C
Equilibrium of humidification: No increase by 0.01 wt% in 5 minutes was confirmed within the range that did not exceed 120 minutes.
Relative humidity program: Increase by 5% RH from 5% RH to 95% RH and decrease by 5% RH from 95% RH to 5% RH

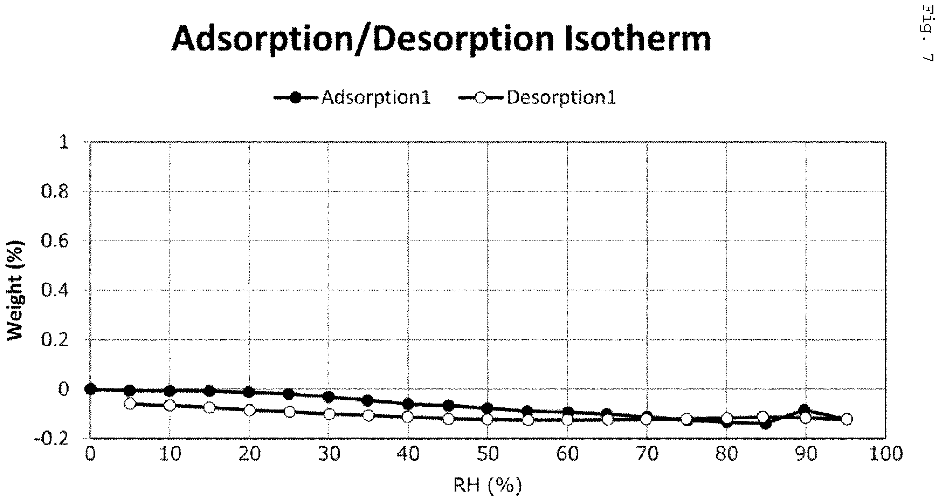

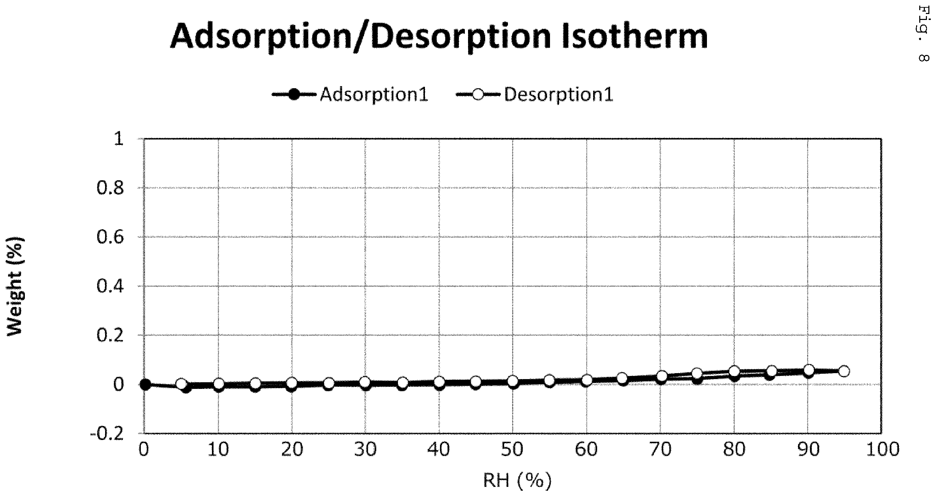

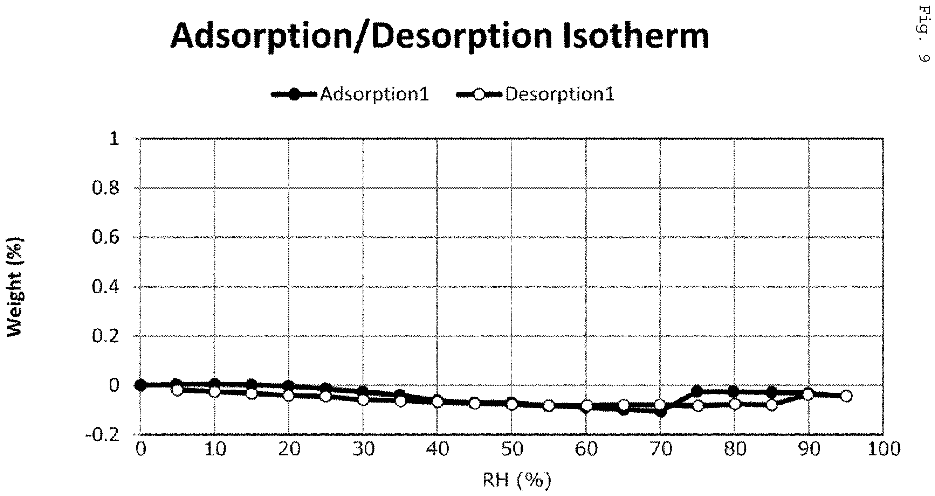

The weight changes in the range of the measurement conditions obtained in these tests are shown in Figs. 7 to 9.

As shown in Figs. 7 to 9, it was clarified that the type I crystal, type II crystal, and type III crystal of compound (I) all showed almost no moisture absorption at 95% relative humidity in the moisture adsorption/desorption test.

Accordingly, the type I crystal, type II crystal, and type III crystal of compound (I) have low moisture absorption and are deemed to be superior in terms of industrial production of pharmaceutical products with stable quality in the development of candidate compounds for pharmaceutical products.

The type I crystal, type II crystal, and type III crystal of compound (I) have high stability as described above. A drug suspension or drug solution of compound (I) can be prepared by adding these crystals, together with an amphiphilic compound, such as hydroxypropyl methylcellulose or dimethyl sulfoxide (DMSO), to water.

### Test Example 3: Pharmacokinetics

The required amount of compound (I) was weighed, and a drug suspension was prepared in 0.5% hydroxypropyl methylcellulose. The number of rats in each group was two, and each drug suspension was administered orally to each male rat (Crl: CD(SD)) using an oral sonde (the dose of the compound was 10 mg/kg). At each blood collection point (0.5, 1, 2, 4, 8, and 24 hours after administration), blood was collected from the jugular vein using a syringe and a needle (the anticoagulant was heparin sodium). The collected blood was centrifuged (13000 rpm, 2 min, 4°C) to prepare plasma, followed by deproteinization. Thereafter, the concentration of the compound in the plasma was measured by LC/MS/MS (LCMS-8040 (Shimadzu Corporation) or API 4000 (AB Sciex), LC: 30-A and 20-A series (Shimadzu Corporation) or Waters Acquity (Waters Corporation)). A drug suspension was prepared and administered, and the concentration of the compound was measured, in the same manner as described above, except that compound A was used in place of compound (I). The results are shown in Table 3.

As shown in Table 3, it was confirmed that compound (I) had an improvement in area under the curve (AUC) of 1900 times or more and an improvement in maximum blood concentration Cₘₐₓ of 1100 times or more as compared to compound A. Accordingly, it was shown that compound (I) had unexpected high pharmacokinetics as compared to compound A, which was a known compound.

**Table 3**

| AUC and Cₘₐₓ of each compound | | |
|---|---|---|
| | AUC_{0-∞} | Cₘₐₓ (µM) |
| Compound A | 0.003 | 0.002 |
| Compound (I) | 5.90 | 2.20 |

### Test Example 4: Evaluation of drug efficacy in diabetes mellitus (DM) rat model

To examine the effect of the compound in a diabetes mellitus model, bladder function was analyzed by cystometry based on the method of Saitoh et al. (European Journal of Pharmacology 501 (2004) 143-149).

A streptozotocin (STZ) solution obtained by dissolving STZ in a citrate buffer solution (0.1 M citric acid, pH of 4.2) to a concentration of 32.5 mg/mL was prepared, and administered intraperitoneally (65 mg/kg) to SD rats to induce DM models. The same procedure was performed on sham rats except that a citrate buffer solution was used in place of the STZ solution.

On the day after the model production and 4 weeks later, a blood sample was taken from the tail vein with the rats awake to measure the blood glucose level. AntSense III (Horiba Corporation) was used for the measurement of blood glucose level. Individuals with a blood glucose level of 300 mg/dL or more on the day after the production of the model and 4 weeks later were determined to be established DM models and used in subsequent operations.

The administration of a test substance (compound A or compound (I)) was started from the day after the production of the model. A vehicle, or a solution for administering the compound was administered orally twice a day, morning and evening, at a volume of 10 mL/kg for 4 weeks. On the day after the last administration of the test substance, an incision was made in the abdomen of each rat under isoflurane anesthesia, and a catheter (PE-90, Becton Dickinson and Company) was inserted through the apex of the bladder for intravesical pressure measurement. After closing up the abdomen, the rats were placed in a holding cage. At least 30 min after the release of isoflurane anesthesia, a urethane solution was administered subcutaneously (0.8 g/kg).

A pressure transducer (DX-360, Nihon Kohden Kogyo Co., Ltd.) and an infusion pump (TE-331S, Terumo Corporation) were connected via a three-way valve to the other end of the catheter inserted into the bladder. Intravesical pressure during continuous intravesical infusion of a saline solution (infusion rate: 12 mL/hr) was measured using a pressure transducer and polygraph (AP-641G, Nihon Kohden Kogyo Co., Ltd.), and recorded continuously on a computer using a PowerLab (ML866, AD Instruments) (sampling rate: 20/sec). The volume of urination was automatically measured using an electronic balance (GX-200, A&D Company Limited) set directly under the holding cage, and recorded continuously on a computer using a PowerLab (sampling rate: 20/sec). Urinary function parameters were recorded and analyzed using PowerLab analysis software Chart 5 (ver. 5.5.6, AD Instruments).

Immediately after urethane anesthesia, continuous intravesical infusion of saline solution (infusion rate: 3 mL/hr) was performed for about 1.5 hours to familiarize the rats with the cystometry environment. After acclimatization to the cystometry environment, urinary function (intravesical bladder pressure, volume of urination, and residual urine volume) was measured by cystometry (infusion rate: 12 mL/hr).

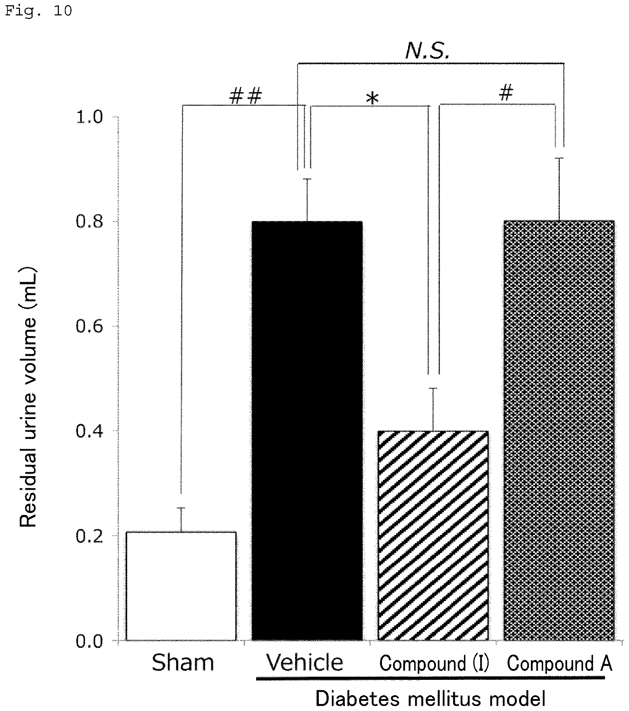

As shown in Fig. 10, compound (I) was found to have remarkable drug efficacy as compared to the compound A.

### Test Example 5: Measurement of nerve growth stimulation effect

To evaluate the nerve growth stimulation effect, neurite bearing assay (proportion of neurite-bearing cells) and neurite length assay (neurite length) were analyzed with reference to the method of Topalli et al. (Brain Research 1030 (2004), pp. 116-124).

An ND3 neuronal cell line (European Collection of Authenticated Cell Cultures) was seeded in a DMEM medium containing 10% fetal bovine serum (0.6 × 10⁴ cells/1 mL) in a 24-well plate. 24 hours later, the medium of seeded cells was removed, and a solution in which compound (I) that was sequentially diluted in dimethyl sulfoxide (DMSO) was adjusted in DMEM containing 0.1% bovine serum albumin so that the DMSO final concentration was 0.1%, was added to cells (evaluation performed in duplicate). After six hours from the addition, the proportion of neurite-bearing cells (the number of cells having a neurite longer than the diameter of the cell body/number of total cells), and the neurite length per cell were analyzed. Further, a test was performed in the same manner as described above, except that compound A was used in place of compound (I). The number of total cells, proportion of neurite-bearing cells, and neurite length per cell were analyzed using a MetaMorph Neurite Outgrowth Application Module (Molecular Devices Japan K.K.). The number of cells was defined as the relative cell number, taking the number of cells per well containing DMSO as 1 (Tables 4 and 5). Table 5 shows the relative activity of compound A when the maximum activity of 3-(15-methoxypentadecyl)-2,4,4-trimethylcyclohex-2-en-1-one (compound (I)) in each test was defined as 1. The test was conducted multiple times, and the average value was shown. Data with a number of cells of 0.5 or more was used. Accordingly, if the number of cells was less than 0.5, the neurite length per cell and the proportion of neurite-bearing cells were defined as "-".

As shown in Table 4, when compound (I) was added in an amount of 1 to 100 µM, the number of relative cells was 0.5 or more.

**Table 4**

| Relative cell number at various concentrations of compound (I) | | | | | | |
|---|---|---|---|---|---|---|
| | Number of cells | | | | | |
| | 1 µM | 3 µM | 5 µM | 10 µM | 30 µM | 100 µM |
| Compound (I) | 1.06 | 1.01 | 0.94 | 0.92 | 0.96 | 0.92 |

As shown in Table 5, it was confirmed that compound (I) had an excellent nerve growth stimulation effect compared to compound A.

**Table 5**

| | Number of cells | | | | | | Neurite length per cell | | | | | | Proportion of neurite-bearing cells | | | | | |
|---|---|---|---|---|---|---|---|---|---|---|---|---|---|---|---|---|---|---|
| | 1 µM | 3 µM | 5 µM | 10 µM | 30 µM | 100 µM | 1 µM | 3 µM | 5 µM | 10 µM | 30 µM | 100 µM | 1 µM | 3 µM | 5 µM | 10 µM | 30 µM | 100 µM |
| Compound A | 1.03 | 0.92 | 0.92 | 1.01 | 0.19 | | 0.26 | 0.33 | 0.39 | 0.71 | - | | 0.24 | 0.34 | 0.41 | 0.70 | - | |

## Claims

1. A crystalline form of 3-(15-methoxypentadecyl)-2,4,4-trimethylcyclohex-2-en-1-one having at least three peaks at diffraction angles (2Θ+0.2°) selected from 7.0°, 14.0°, 17.5°, 19.5°, 21.0°, 23.7°, and 24.8° in a powder X-ray diffraction spectrum (CuKα).

2. The crystalline form according to claim 1, wherein the crystalline form has peaks at diffraction angles (2Θ+0.2°) of 7.0°, 14.0°, 17.5°, 19.5°, 21.0°, 23.7°, and 24.8° in a powder X-ray diffraction spectrum (CuKα).

3. The crystalline form according to claim 1 or 2, wherein the powder X-ray diffraction spectrum is substantially the same as the spectrum shown in Fig. 1.

4. The crystalline form according to any one of claims 1 to 3, wherein the crystalline form has an endothermic peak at a peak temperature of around 35°C in differential scanning calorimetry (DSC measurement).

5. A pharmaceutical composition comprising the crystalline form of any one of claims 1 to 4.
